# EUROPEAN PATENT SPECIFICATION

(11) **EP 1 276 441 B1**
(45) Date of publication and mention of the grant of the patent: **23.03.2005**
(21) Application number: 00931753.8
(22) Date of filing: 31.05.2000
(51) Int. Cl.: A61F 2/62

(54) **LEG PROSTHESIS**
BEINPROTHESE
PROTHESE DE JAMBE

(30) Priority: 03.06.1999 NO 992689
(43) Date of publication of application: 22.01.2003
(73) Proprietor: In-Lieu AS, 7013 Trondheim (NO)
(72) Inventor: Andersen, Björn, 6003 Aalesund (NO)
(74) Representative: Dealtry, Brian
(86) International application number: PCT/NO2000/000185
(87) International publication number: WO 2000/074610

(56) References cited:
- EP-A1- 0 728 451
- WO-A1-98/29059
- FR-A1- 2 770 772
- US-A- 4 883 493
- US-A- 5 702 488

## Description

The invention relates to a device according to the preamble of claim 1, for height control of a leg prosthesis.

### Background

Following amputation of a leg, at the calf, knee, thigh or hip, a patient often receives a prosthesis as a substitute for the lost body part. The remaining of the patient is generally connected to the prosthesis by a sleeve, named "bit-sleeve". It is also possible to connect the prosthesis directly to the remaining body part with a titanium screw, so-called osseo-integration or tissueintegrated prosthesis. The bit-sleeve is provided with an adaptor and an ankle connected to a foot piece. This kind of leg prosthesis may also comprise a knee and a hip, dependent on the amputation level.

A leg prosthesis provided with telescopic regulative parts, to make height controls due to different conditions, is well known. E.g. from Swedish patent publication 431.054 (LIC) such a leg prosthesis with telescopic control in the leg part, is known. This control requires that a locking device is unfastened. This is time consuming, and not fit for adjustment while walking.

From US patent publication 5,376,138 (Bouchard *et al*) it is known to use a hydraulic shock absorber to absorb shock against a leg prosthesis. This requires a complicated cylinder and valve arrangement, in order to absorb quick shocks, e.g. upon a fall, without comprising the shock absorbation, and in addition, provide locking, e.g. for walking stairs. This solution is also not very suitable, for use where it is desirable for a fast and simple height control, e.g. a short walk on sloping ground.

US patent publication 5,702,488 (Wood *et al*) describes yet another telescopic shock absorber for a leg prosthesis, without the possibility to control height.

It is difficult for people with an amputation, especially a thigh amputation, to walk on sloping and/or rugged ground. This is also true for walking on pavements having a slope towards the street. Where there is a small slope one can compensate by using the healthy leg, but this can only be carried out over short distances and demands that the slope is in a predetermined direction.

### Object

The main object of the invention is to create a device for height control, which at the same time can function as a shock absorber, where this is desirable and practical. It should be manually easy to activate, preferably without visible encroachment. It should be made of simple parts, preferably elements that are commercially available.

### The invention

The invention is defined in patent claim 1. With such a device it is possible to perform quick control and fine adjustment of the prosthesis height, in such a way that it is possible to adapt the prosthesis for the objects mentioned above. This may take place without effecting the shock absorbing properties of the prosthesis. Disengagement may be achieved in a simple way, adjusted to the needs and desires of the prosthesis user.

An important advantage of the invention, is that the activation needed to initiate the height control can, if desirable, be done without direct hand operation, as a valve can be opened upon a single press with the healthy foot

Patent claims 2 to 10 relates to several details of the invention. The construction and function of the invention will be described further in the example below.

### Example

The invention will be described hereinafter with reference to the accompanying drawings, wherein:
Figure 1 shows a sectioned side view of an embodiment of the invention, for placing in the calf area of the leg prosthesis, and
Figure 2 shows the principles of the piston of Figure 1.

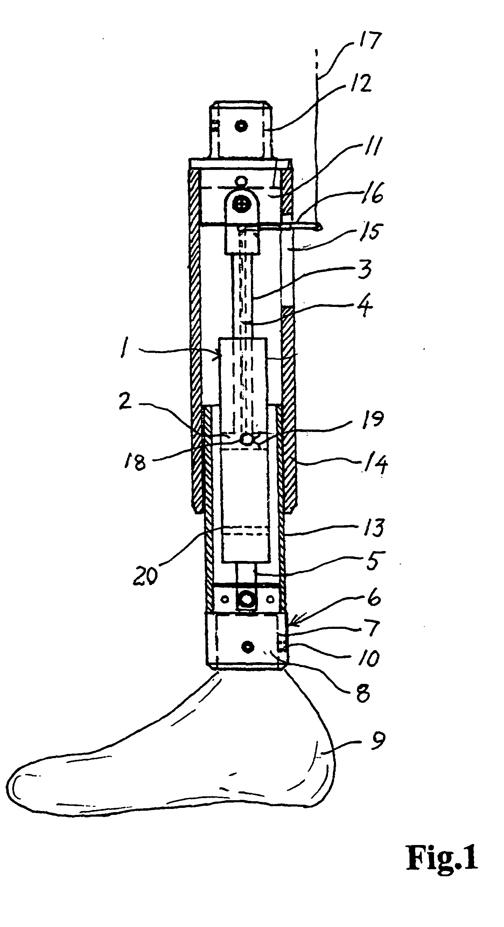

The device according to Figure 1, comprises a cylinder 1 with a piston 2, the piston has a piston rod 3, which is led vertically upwards through the upper end wall of the cylinder. A suitable gas, e.g. nitrogen, is filled into the cylinder 1, on both sides of the piston 2.

To make the neutral or rest-position of the piston 2 adjustable, a valve 18 is provided, with connections to both sides of the piston 2. The valve 18 is normally closed, but can be opened via spring tension with an activation lever 4, which is led through the piston rod 3.

The lower end of the cylinder 1 is provided with a protruding pin 5, carrying a connection head 6. The connection head has at it's downward end a cup 7 for receiving a pin 8 protruding from a foot piece 9. Lock screws 10 in threaded holes are used for locking of the foot piece 9 to the connection head 6.

Correspondingly, the piston rod carries an upper connection head 11 with an upward cup 12 for receiving a pin or plug (not shown) attached to the upper portions of the leg prosthesis. The lower connection head 6 has a protruding, inner telescopic tube 13, which in all possible positions of the gas spring, overlaps with an upper outer telescopic tube 14 attached to the upper connection head. The telescopic tubes 13 and 14 may be of plastic, especially reinforced with carbon fibre or light metal, particularly titanium.

The upper outer telescopic tube 14 has an axial slit 15 at the upper end, for receiving a release lever 16 connected to the activation lever 4. The release lever 16 can be arranged to open the valve 18 upon either downwards or upwards pressure. In the illustrated example it is made as a double lever, so that it opens the valve 18 upon an upward pull on a string 17. In the illustrated example, string 17 is shown exterior to the leg prosthesis, however it can also be hidden with in the leg prosthesis.

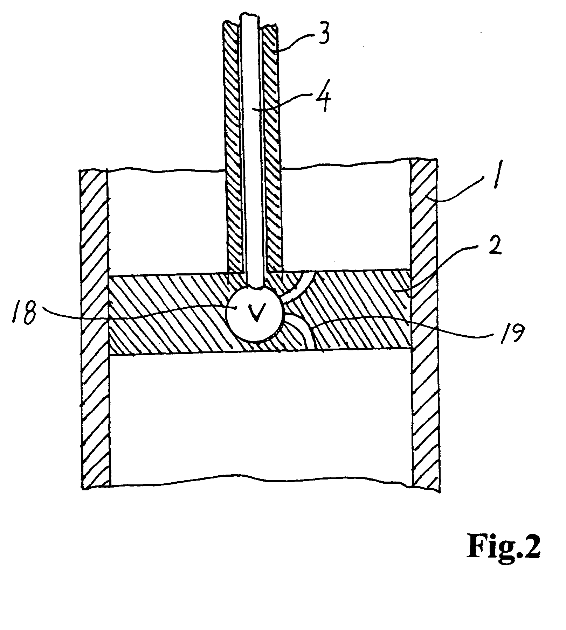

Figure 2 shows the details of a piston 2 with valve 18, that can be opened upon pressure from the activation lever 4 which is protruding through the piston rod 3. Upon opening of the valve 18, pressure medium can flow through a canal 19, which communicates both sides of the piston 2.

In order to lengthen the leg prosthesis, the user opens the valve 18, and shifts weight to the healthy foot, so that the leg prosthesis is unloaded. Thereby, the piston will be expanded, in the illustrated example because the side of the piston where the piston rod is attached, has a lower pressure areal. In addition, the piston may be provided with a screw spring or gas spring on the opposite side, increasing the expansion. The gas spring may for example comprise an extra piston 20 (Figure 1) in the inner of the cylinder, enclosing a gas pillow. This is particularly pertinent when the area of the piston rod is too small to provide sufficient pressure difference.

The area difference between the two sides of the piston, the pressure in the cylinder, the area of the canal 19, and the size of an eventual additional prestressing, will control the speed of the expansion. This speed can thereby be determined accordingly, by choosing suitable dimension of these elements.

To shorten the leg prosthesis, the user must, with the valve open, load the leg prosthesis with weight, until the piston 2 is pushed into the cylinder 1, to the desired degree. Then the valve closes.

Upon an alternative, not illustrated embodiment, the activation lever 4 can be connected to an electrical relay, which is dimensioned to open the valve, and that can receive power from a battery carried on the body of the user, through a key-switch or micro-switch placed suitable for activation from the user of the prosthesis.

Upon walking normally with the leg prosthesis, the compressible medium, meaning the gas inside the cylinder, will function as a shock absorber. The pressure in the shock absorbing gas can be varied, so that the shock absorbing characteristic can be adjusted according to need, dependent of the weight of the person etc.

It is also possible to make a cylinder-piston device with an incompressible medium, for example hydraulic oil or gas under high pressure, and to provide the shock absorbing by means of a separate compressible piece of material, or a separate screw spring or gas pillow connected to the cylinder. The shock absorbation can be set at a low level, so that it functions as a security when jumping from stairs etc. Then it will prevent too high a pressure on the amputated body part and vertebral column.

### Use and function

When the user of the prosthesis wishes to lengthen the leg prosthesis, the valve 18 ergo opens and the healthy leg becomes loaded, at the same time as the leg prosthesis becomes unloaded. The piston 2 will then expand towards an end position, by the overpressure in one part of the chamber, eventually supported by extra prestressing as described above. The piston 2 may then be stopped in a random position, where a pressure balance is established between the two sides of the piston. In this way, it becomes possible to carry out a stepless height control of a leg prosthesis, without conspicuous and time-consuming activation. By suitably dimensioning the canal 19 in the piston, and the rest of the elements mentioned above, a lengthening will occur so slowly that the prosthesis user easily can decide the degree of lengthening.

Likewise, the user can open the valve 18 and load the leg prosthesis to press the piston 2 further into the cylinder 1 until desired length is achieved.

The control mechanism, meaning the element activating the valve opening, and thereby the length control trigger, can be placed where most convenient according to the amputation level. If the person has been hip-operated, the control mechanism can be placed on the prosthesis at thigh height

It is also possible to place such a trigger mechanism at foot height, so that the string or wire from a micro switch ascends through the tubes 13 and 14.

In stead of leading the canal between the two sections of the cylinder, through the piston, it is possible, in order to save inner space, to make a canal in the cylinder wall, or with a pipe or tube connecting the two ends of the cylinder with each other. In such a canal, pipe or tube, a valve is then installed with the same function as the valve 18 in the example given above.

The device can be provided with different forms of controlled regulation, either stepless or in fixed steps, for example 0,5, 1, 2 cm. This may also be done by means of a service device on a control panel. It is also possible to make an electronic control which is programmed for adjusting different prosthesis levels. Such a control may be stepless with a remote control. Regulation of the shock absorbation can also be placed in this system. A presetting for the shock absorbation to different degrees, may be made.

By programming, it is possible to adjust the prosthesis to different ranges of application, for example different walking paths. This means that the prosthesis will perform the level adjustments corresponding to the current distance, for example from home to work, in the i case where this is a walking distance. One alternative is to program for walking across different types of terrain, so that the "walk mode" can be activated manually when desired. Such a program can be co-ordinated with regulations of the shock absorbency, so that it is adjustable for the current terrain.

This control may be achieved either by service elements on the prosthesis, or with a a remote control in a similar way to a remote control for locks. The adjustment or programming can then be done in a microprocessor on the prosthesis, activated by a remote control with one or more channels of the known type.

It is also possible to adjust the level regulation so that it is triggered by a pressure sensor. One may then arrange the control so that the mean pressure over time, corresponding to 5 walking on flat and stable ground, becomes the reference level. Upon deviation from this pressure, for example upon walking on rough ground, a regulation can be done according to the pressure. This regulation must be done gradually over a few steps, if it should be regulated more than 0,5 cm. The user then has the ability to store determined pressure-patterns electronically, so that the prosthesis retrieves a certain program segment when this is registered at the pressure sensor. Such an activation could be controlled with a delay function. In its most advanced state of automation, the prosthesis may be provided with an ultrasound- transmitter/receiver or another wave based distance measurement, that can register the oncoming terrain in the walking Path, and in this way send a signal for activating a certain program, and thereby level-adjustment.

## Claims

1. Device for height control of a leg prosthesis, comprising a cylinder (1) with a piston (2) and a piston rod (3), said piston rod (3) being led out of one end of the cylinder, the cylinder and the piston rod each being connected to one of two connecting parts (6, 11), which again may be connected to parts of the leg prosthesis, and the cylinder containing a pneumatic or hydraulic medium,
**characterized in that** in connection with the cylinder (1), there is a canal for transferring a medium from one side of the piston to the other, through a valve (18) that can be operated by the prosthesis user, the piston (2) being prestressed against an outer end position, by virtue of differential pressure areal between the two sides of the piston.

2. Device according to claim 1,
**characterized in that** one side of the piston is prestressed towards an outer end position with a helical spring or a gas spring.

3. Device according to patent claim 1 or 2,
**characterized in that** the canal (19) and the valve (18) located within the piston (2), with an activation device (4) led through the piston rod (3).

4. Device according to any one of patent claims 1-3, with an incompressible medium or a gas under high pressure in the cylinder,
**characterized in that** in addition to the incompressible medium, there is a gas volume in the cylinder, or a compressible element, or a helical spring, or a gas pillow, in or adjacent to the cylinder, capable of absorbing shock.

5. Device according to patent claim 1,
**characterized in that** the canal for transferring pneumatic or hydraulic medium from one part of the cylinder to the other, is in the cylinder wall, or a pipeline exterior to this, and the valve is located externally to the cylinder.

6. Device according to patent claim 3,
**characterized in that** the activation device (4) is connected to a release lever (16) that may be moved by a string (17) which is led up through the prosthesis, to the knee or thigh area of the prosthesis user, or down to the foot-area.

7. Device according to anyone of the patent claims 1-6,
**characterized in that** the cylinder is surrounded by a telescopic tube (14) protruding over the cylinder from the free end of the piston rod.

8. Device according to patent claim 7,
**characterized in that** the telescopic tube is comprised of two tubes (13, 14), one extending from each end of the device, the tubes are arranged for sliding movement towards each other.

9. Device according to patent claim 1,
**characterized in that** the valve is provided with an electrical tripping relay, which may be activated from a micro switch.

10. Device according to any of the patent claims 1-9,
**characterized in that** an end of the cylinder and a free end of the piston rod, are provided with a connection piece (6, 11), having a cup-shaped opening at the end, for receiving the end of a prosthesis part.

11. Device according to anyone of the patent claims 1-10,
**characterized in that** the valve (18) may be operated through an electric circuit, that may be controlled by remote control, and that alternatively may be programmed for manual or automatic connection to predetermined level-controlling programs.

12. Device according to patent claim 11,
**characterized in that** the electronic circuit receives control signals from a pressure sensor.

13. Device according to patent claim 11 or 12,
**characterized in that** it comprises a wave-based distance meter, registering the ground of the walking path, as basis for influencing the electric circuit.

14. Device according to anyone of the patent claims 11-13,
**characterized in that** it comprises a wireless coupling mechanism for connecting programmed functions.

15. Device according to anyone of the patent claims 11-14,
**characterized in that** the control of the prosthesis height level is coordinated with a control of the prosthesis' shock absorbency.

## Patentansprüche

1. Vorrichtung zur Höhensteuerung einer Beinprothese mit einem Zylinder (1) mit einem Kolben (2) und einer Kolbenstange (3), wobei die Kolbenstange (3) aus einem Ende des Zylinders herausgeführt ist, der Zylinder und die Kolbenstange jeweils mit einem von zwei Verbindungsteilen (6,11) verbunden sind, die wiederum mit Teilen der Beinprothese verbunden sein können, und der Zylinder ein pneumatisches oder hydraulisches Medium enthält,
**dadurch gekennzeichnet, dass** in Verbindung mit dem Zylinder (1) ein Kanal zum Übertragen eines Mediums von einer Seite des Kolbens zur anderen über ein Ventil (18), das durch den Prothesenbenutzer betätigt werden kann, besteht, wobei der Kolben (2) gegen eine äußere Endposition mittels eines Differentialdruckbereichs zwischen den beiden Seiten des Kolbens vorbelastet ist.

2. Vorrichtung nach Anspruch 1, **dadurch gekennzeichnet, dass** eine Seite des Kolbens zu einer äußeren Endposition mit einer Spiral- bzw. Schraubenfeder oder einer Gasfeder vorbelastet ist.

3. Vorrichtung nach Anspruch 1 oder 2, **dadurch gekennzeichnet, dass** der Kanal (19) und das Ventil (18) sich in dem Kolben (2) befinden, wobei eine Aktivierungsvorrichtung (4) durch die Kolbenstange (3) geführt ist.

4. Vorrichtung nach einem der Ansprüche 1 bis 3, mit einem inkompressiblen Medium oder einem Gas unter hohem Druck in dem Zylinder, **dadurch gekennzeichnet, dass** zusätzlich zu dem inkompressiblen Medium ein Gasvolumen in dem Zylinder oder ein komprimierbares Element oder eine Spiral- bzw. Schraubenfeder oder ein Gaspolster in oder benachbart dem Zylinder vorgesehen ist, das in der Lage ist, einen Stoß aufzunehmen bzw. zu dämpfen.

5. Vorrichtung nach Anspruch 1, **dadurch gekennzeichnet, dass** der Kanal zum Übertragen des pneumatischen oder hydraulischen Mediums von einem Teil des Zylinders zum anderen sich in der Zylinderwand oder einer außerhalb dieser befindlichen Rohrleitung befindet, und das Ventil sich außerhalb des Zylinders befindet.

6. Vorrichtung nach Anspruch 3, **dadurch gekennzeichnet, dass** die Aktivierungsvorrichtung (4) mit einem Freigabehebel (₁6) verbunden ist, der durch eine Schnur bzw einen Zug (17), die/der durch die Prothese nach oben zum Knie- oder Schenkelbereich des Prothesenbenutzers oder nach unten zum Fußbereich geführt ist, bewegt werden kann.

7. Vorrichtung nach einem der Ansprüche 1 bis 6, **dadurch gekennzeichnet, dass** der Zylinder von einem Teleskoprohr (14) umgeben ist, das über den Zylinder von dem freien Ende der Kolbenstange vorsteht.

8. Vorrichtung nach Anspruch 7, **dadurch gekennzeichnet, dass** das Teleskoprohr aus zwei Rohren (13,14) besteht, von denen sich je eines von jedem Ende der Vorrichtung erstreckt, wobei die Rohre für eine Gleitbewegung aufeinander zu angeordnet sind.

9. Vorrichtung nach Anspruch 1, **dadurch gekennzeichnet, dass** das Ventil mit einem elektrischen Auslöserelais versehen ist, das von einem Mikroschalter betätigt werden kann.

10. Vorrichtung nach einem der Ansprüche 1 bis 9, **dadurch gekennzeichnet, dass** ein Ende des Zylinders und ein freies Ende der Kolbenstange mit einem Verbindungsstück (6,11) mit einer napfförmigen Öffnung an dem Ende zur Aufnahme des Endes eines Prothesenteils versehen sind.

11. Vorrichtung nach einem der Ansprüche 1 bis 10, **dadurch gekennzeichnet, dass** das Ventil (18) über eine elektrische Schaltung betätigt werden kann, die durch Fernsteuerung gesteuert werden kann, und die alternativ für eine manuelle oder automatische Verbindung zu vorbestimmten Höhensteuerungsprogrammen programmiert sein kann.

12. Vorrichtung nach Anspruch 11, **dadurch gekennzeichnet, dass** die elektronische Schaltung Steuersignale von einem Drucksensor empfängt.

13. Vorrichtung nach Anspruch 11 oder 12, **dadurch gekennzeichnet, dass** sie einen auf Wellen basierenden Entfernungsmesser umfasst, der den Boden des Gehwegs als Basis zur Beeinflussung der elektrischen Schaltung abtastet.

14. Vorrichtung nach einem der Ansprüche 11 bis 13, **dadurch gekennzeichnet, dass** sie einen drahtlosen Koppelungsmechanismus zum Verbinden programmierter Funktionen aufweist.

15. Vorrichtung nach einem der Ansprüche 11 bis 14, **dadurch gekennzeichnet, dass** die Steuerung des Prothesen-Höhenniveaus mit einer Steuerung der Stoßdämpfung der Prothese koordiniert ist bzw. wird.

## Revendications

1. Dispositif pour commander la hauteur d'une prothèse de jambe, comprenant un vérin (1) muni d'un piston (2) et d'une tige de piston (3), ladite tige de piston (3) étant entraînée hors d'une extrémité du vérin, le vérin et la tige de piston étant chacun relié à l'une de deux pièces de liaison (6, 11), qui peuvent également être reliées à des pièces de la prothèse de jambe, et le vérin contenant un milieu pneumatique ou hydraulique,
**caractérisé en ce qu'**il existe un canal en liaison avec le vérin (1) destiné à transférer un milieu d'un côté du piston à l'autre, à travers une vanne (18) qui peut être actionnée par l'utilisateur de la prothèse, le piston (2) étant précontraint contre un emplacement d'extrémité externe, grâce à une pression différentielle aréolaire entre les deux côtés du piston.

2. Dispositif selon la revendication 1,
**caractérisé en ce qu'**un côté du piston est précontraint contre un emplacement d'extrémité externe à l'aide d'un ressort hélicoïdal ou d'un ressort à gaz.

3. Dispositif selon la revendication 1 ou 2,
**caractérisé en ce que** le canal (19) et la vanne (18) sont situés à l'intérieur du piston (2), un dispositif d'actionnement (4) étant entraîné à travers la tige de piston (3).

4. Dispositif selon l'une quelconque des revendications 1 à 3, doté d'un milieu incompressible ou d'un gaz sous pression élevée dans le vérin,
**caractérisé en ce que**, outre le milieu incompressible, il existe un volume de gaz à l'intérieur du vérin, ou bien un élément compressible ou un ressort hélicoïdal ou encore un coussin de gaz à l'intérieur du vérin ou de manière adjacente au vérin, capable d'absorber les chocs.

5. Dispositif selon la revendication 1,
**caractérisé en ce que** le canal destiné à transférer le milieu pneumatique ou hydraulique d'une partie du vérin à l'autre se trouve dans la paroi de vérin ou dans un conduit extérieur à celui-ci, et la vanne se situe à l'extérieur du vérin.

6. Dispositif selon la revendication 3,
**caractérisé en ce que** le dispositif d'actionnement (4) est relié à un levier de déverrouillage (16) qui peut être déplacé au moyen d'un câble (17) entraîné vers le haut à travers la prothèse, jusqu'à la zone du genou ou de la hanche de l'utilisateur de la prothèse, ou vers le bas jusqu'à la zone du pied.

7. Dispositif selon l'une quelconque des revendications 1 à 6,
**caractérisé en ce que** le vérin est entouré d'un tube télescopique (14) dépassant au-dessus du vérin hors de l'extrémité libre de la tige de piston.

8. Dispositif selon la revendication 7,
**caractérisé en ce que** le tube télescopique se compose de deux tubes (13, 14), chacun partant d'une extrémité du dispositif, les tubes étant disposés afin de pouvoir se déplacer en coulissant l'un vers l'autre.

9. Dispositif selon la revendication 1,
**caractérisé en ce que** la vanne est équipée d'un relais déclencheur électrique, qui peut être activé à l'aide d'un micro-commutateur.

10. Dispositif selon l'une quelconque des revendications 1 à 9,
**caractérisé en ce qu'**une extrémité du vérin et une extrémité libre de la tige de piston sont équipées d'une pièce de liaison (6, 11), dont l'extrémité est munie d'une ouverture en forme d'entonnoir, afin de recevoir l'extrémité d'une pièce de prothèse.

11. Dispositif selon l'une quelconque des revendications 1 à 10,
**caractérisé en ce que** la vanne (18) peut être actionnée par l'intermédiaire d'un circuit électrique, qui peut être télécommandé ou, comme variante, être programmé pour établir une liaison manuelle ou automatique avec des programmes prédéfinis de commande de niveau.

12. Dispositif selon la revendication 11,
caractérisé en que le circuit électronique reçoit des signaux de commande d'un capteur de pression.

13. Dispositif selon la revendication 11 ou 12,
**caractérisé en ce qu'**il comprend un télémètre à ondes enregistrant le terrain du chemin de marche en tant que base pour influencer le circuit électrique.

14. Dispositif selon l'une quelconque des revendications 11 à 13,
**caractérisé en ce qu'**il comprend un mécanisme d'accouplement sans fil afin de relier les fonctions programmées.

15. Dispositif selon l'une quelconque des revendications 11 à 14,
**caractérisé en ce que** la commande de niveau de hauteur de la prothèse est coordonnée à une commande d'absorption des chocs subis par la prothèse.
